# EUROPEAN PATENT APPLICATION

(11) **EP 4 700 781 A1**
(43) Date of publication of application: **25.02.2026**
(21) Application number: 25195886.4
(22) Date of filing: 14.08.2025
(51) Int. Cl.: G16H 10/60, G16H 50/70, G16B 20/00, G16B 45/00, G16B 50/30

(54) **CONVEYING INFORMATION ABOUT COMPATIBILITY OF A DONOR AND RECEIPIENT OF A TRANSPLANT**

(30) Priority: 20.08.2024 US 202418810034; 18.09.2024 LU 508295
(71) Applicant: Immucor GTI Diagnostics, Inc., Waukesha, WI 53186 (US)
(72) Inventor: Jacobson, Emma, Norwalk (US); Limauro, Jeffrey, Cheshire (US); DeConinck, Martha, Southbury (US); Zheng, Yaohua, Brookfield (US); Christy, Thomas, Wauwatosa (US)
(74) Representative: Müller-Boré & Partner Patentanwälte PartG mbB

(57) **Abstract**

An example method includes obtaining information about a recipient of a transplant and a potential donor of the transplant, where the information includes an HLA (human leukocyte antigen) typing for the recipient and an HLA typing for the potential donor; obtaining multiple datasets based on the information, where each dataset includes indicators that are based on a compatibility of the potential donor to the recipient for the transplant; storing the multiple data sets in database; generating data for a user interface (UI) containing representations of the indicators from ones of the multiple datasets in the database; and outputting the data for display on a display device.

## Description

### TECHNICAL FIELD

This specification relates generally to example systems and processes for conveying information about compatibility of a donor and a recipient of a transplant.

### BACKGROUND

A person who receives a transplant, such as stem cells or a solid organ (e.g., a kidney or liver), is referred to as the transplant recipient or simply recipient. A person who donates the transplant is referred to as the transplant donor or simply donor.

The more compatible the donor and the recipient are, the less likely that the recipient will reject the transplant. One measure of compatibility is based on the HLA (human leukocyte antigen) typings of the recipient and the donor. Various techniques may be used to determine recipient/donor compatibility based on the HLA typings. These techniques may each provide a different type of indicator to specify the level of compatibility between the donor and the recipient.

### SUMMARY

An example method is performed by one or more processing devices and includes the following operations: obtaining information about a recipient of a transplant and a potential donor of the transplant, where the information includes an HLA (human leukocyte antigen) typing for the recipient and an HLA typing for the potential donor; obtaining multiple datasets based on the information, where each dataset includes indicators that are based on a compatibility of the potential donor to the recipient for the transplant; storing the multiple data sets in database; generating data for a user interface (UI) containing representations of the indicators from ones of the multiple datasets in the database; and outputting the data for display on a display device. The method may include one or more of the following features, either alone or in combination.

An further example method is performed by one or more processing devices and includes the following operations: providing information about a recipient of a transplant and a potential donor of the transplant, where the information includes an HLA (human leukocyte antigen) typing for the recipient and an HLA typing for the potential donor; obtaining and/or providing multiple datasets based on the information, where each dataset includes indicators that are based on a compatibility of the potential donor to the recipient for the transplant; storing the multiple data sets in database; generating data for a user interface (UI) containing representations of the indicators from ones of the multiple datasets in the database; and outputting the data for display on a display device. The method may include one or more of the following features, either alone or in combination.

The indicators may be based on compatibility of thymus (T-) cells in the donor to T-cells in the recipient. Alternatively or additionally, the indicators may be based on compatibility of bursa (B-) cells in the donor to B-cells in the recipient. Alternatively or additionally, the indicators may be based on unaligned peptides between the HLA typing of the recipient and the HLA typing of the donor.

The datasets may be from different sources. The different sources may include a local source and a remote source. The local source may include an entity from which the information was obtained. The remote source may be different from the entity from which the patient information was obtained.

Obtaining the information may include receiving results of HLA typing assays performed on biological samples from the recipient and the potential donor.

Providing the information may include receiving results of HLA typing assays performed on biological samples from the recipient and the potential donor.

The information as obtained and/or provided may include results of HLA typing assays of the recipient and the potential donor. The HLA typing assays may be performed on biological samples from the recipient and the potential donor.

The UI may include a table. The table may include columns for the recipient and columns for the potential donor. The columns for the recipient may include a first column containing loci and a second column containing alleles of the recipient at corresponding loci in the first column. The columns for the potential donor may include a third column containing alleles of the potential donor at corresponding loci in the first column and one or more additional columns containing indicators that are based one or more of a compatibility of thymus (T-) cells in the donor to T-cells in the recipient or a compatibility of bursa (B-) cells in the donor to B-cells in the recipient. The one or more additional indicators may be at rows corresponding to loci and alleles of the recipient or rows corresponding to one or more serotype groups of the recipient.

The indicators may include values representing compatibility of the potential donor to the recipient for the transplant. The UI may include a box plot of a population that includes the recipient. The box plot may represent a range of the values for the population that are based on compatibility of members of the population to the recipient for the transplant. Values for multiple potential donors may be overlaid on the box plot. The values for multiple potential donors may be based on compatibility of the multiple potential donors to the recipient for the transplant.

The multiple datasets may include MFI (mean fluorescence intensity) values. The MFI values may be based on antigens of the recipient that are indicative of rejection of the transplant from the potential donor. The data for the UI may include a bar chart containing the MFI values associated with the antigens of the recipient. The bar chart may include first axes for the MFI values and indicators and a second axis corresponding to antigens of the recipient that are indicative of rejection of the transplant from the potential donor.

The UI may include a single window that displays all of the indicators or that is configured for scrolling to reach all indicators.

Storing the multiple data sets in the database may include storing each dataset in a table. Each of the tables may include common information including an identity of the recipient, an identity of the potential donor, an HLA typing of the recipient, and an HLA typing of the potential donor.

One or more non-transitory machine-readable storage devices may store instructions that are executable by one or more processing devices to perform the following operations: obtaining information about a recipient of a transplant and a potential donor of the transplant, where the information includes an HLA (human leukocyte antigen) typing for the recipient and an HLA typing for the potential donor; obtaining multiple datasets based on the information, where each dataset includes indicators that are based on a compatibility of the potential donor to the recipient for the transplant; storing the multiple data sets in database; generating data for a user interface (UI) containing representations of the indicators from ones of the multiple datasets in the database; and outputting the data for display on a display device. One or more of the following features may be included in the foregoing operations.

One or more non-transitory machine-readable storage devices may store instructions that are executable by one or more processing devices to perform the following operations: providing information about a recipient of a transplant and a potential donor of the transplant, where the information includes an HLA (human leukocyte antigen) typing for the recipient and an HLA typing for the potential donor; obtaining and/or providing multiple datasets based on the information, where each dataset includes indicators that are based on a compatibility of the potential donor to the recipient for the transplant; storing the multiple data sets in database; generating data for a user interface (UI) containing representations of the indicators from ones of the multiple datasets in the database; and outputting the data for display on a display device. One or more of the following features may be included in the foregoing operations.

The indicators may be based on compatibility of thymus (T-) cells in the donor to T-cells in the recipient. Alternatively or additionally, the indicators may be based on compatibility of bursa (B-) cells in the donor to B-cells in the recipient. Alternatively or additionally, the indicators may be based on unaligned peptides between the HLA typing of the recipient and the HLA typing of the donor.

The datasets may be from different sources. The different sources may include a local source and a remote source. The local source may include an entity from which the information was obtained. The remote source may be different from the entity from which the patient information was obtained.

Obtaining the information may include receiving results of HLA typing assays performed on biological samples from the recipient and the potential donor.

Providing the information may include receiving results of HLA typing assays performed on biological samples from the recipient and the potential donor.

The information as obtained and/or provided may include results of HLA typing assays of the recipient and the potential donor. The HLA typing assays may be performed on biological samples from the recipient and the potential donor.

The UI may include a table. The table may include columns for the recipient and columns for the potential donor. The columns for the recipient may include a first column containing loci and a second column containing alleles of the recipient at corresponding loci in the first column. The columns for the potential donor may include a third column containing alleles of the potential donor at corresponding loci in the first column and one or more additional columns containing indicators that are based one or more of a compatibility of thymus (T-) cells in the donor to T-cells in the recipient or a compatibility of bursa (B-) cells in the donor to B-cells in the recipient. The one or more additional indicators may be at rows corresponding to loci and alleles of the recipient or rows corresponding to one or more serotype groups of the recipient.

The indicators may include values representing compatibility of the potential donor to the recipient for the transplant. The UI may include a box plot of a population that includes the recipient. The box plot may represent a range of the values for the population that are based on compatibility of members of the population to the recipient for the transplant. Values for multiple potential donors may be overlaid on the box plot. The values for multiple potential donors may be based on compatibility of the multiple potential donors to the recipient for the transplant.

The multiple datasets may include MFI (mean fluorescence intensity) values. The MFI values may be based on antigens of the recipient that are indicative of rejection of the transplant from the potential donor. The data for the UI may include a bar chart containing the MFI values associated with the antigens of the recipient. The bar chart may include first axes for the MFI values and indicators and a second axis corresponding to antigens of the recipient that are indicative of rejection of the transplant from the potential donor.

The UI may include a single window that displays all of the indicators or that is configured for scrolling to reach all indicators.

Storing the multiple data sets in the database may include storing each dataset in a table. Each of the tables may include common information including an identity of the recipient, an identity of the potential donor, an HLA typing of the recipient, and an HLA typing of the potential donor.

At least part of the systems, and processes described in this specification may be executed on, or controlled by, executing on one or more processing devices instructions that are stored on one or more non-transitory machine-readable storage media. Examples of non-transitory machine-readable storage media include read-only memory, an optical disk drive, memory disk drive, and random access memory (RAM). At least part of the systems, and processes described in this specification may be implemented or controlled using a computing system comprised of one or more processing devices and memory storing instructions that are executable by the one or more processing devices to perform various control operations. The systems, and processes described in this specification may be configured, for example, through design, construction, formulation, arrangement, placement, programming, operation, activation, deactivation, and/or control.

Two or more of the features described in this specification, including in this summary section, may be combined to form implementations not specifically described in this specification.

The details of one or more implementations are set forth in the accompanying drawings and the description below. Other features and advantages will be apparent from the description and drawings, and from the claims.

### DESCRIPTION OF THE DRAWINGS

Fig. 1 is a flowchart showing operations included in an example process for conveying information about compatibility of a donor and a recipient of a transplant.
Fig. 2 is a block diagram illustrating, conceptually, how datasets containing indicators of compatibility are obtained from different sources.
Fig. 3 is an example user interface (UI) for providing information to send to sources to obtain the datasets.
Fig. 4 is an example table of a UI showing indicators of compatibility from different sources displayed side-by-side for different donors.
Fig. 5 is another example table of a UI showing indicators of compatibility from different sources displayed side-by-side for different donors.
Fig. 6 is an example box plot showing indicators of compatibility for potential donors displayed relative to a population containing the recipient.
Fig. 7 is an example bar chart that combines mean fluorescence intensity (MFI) values with indicators of compatibility from one or more other sources.

Like reference numerals indicate like elements.

### DETAILED DESCRIPTION

Described herein are examples of systems and processes for conveying information about the compatibility of a recipient of a transplant and potential donors for the transplant. For example, the recipient may need a stem cell transplant or a solid organ transplant, such as a new kidney or a new liver. The systems and processes use information about the recipient and the potential donors to obtain datasets. The datasets include indicators, such as those described herein, that are based on, and may be indicative of, compatibility of potential donors to the recipient. The datasets may also include other information, such as factors on which the compatibility is based.

The datasets may be obtained from multiple sources. For example, the datasets may be obtained from multiple third party services, retrieved from storage that is local or remote, or generated locally. The datasets are stored in a database in the manner described herein so that all or part of the datasets are linked and can be retrieved at any time and displayed on a user interface (UI).

Indicators and, in some cases, other information, from different datasets for different donors may be displayed in the UI alongside each other and alongside information about the recipient. The UI may be or include a single window that displays all of the indicators or that is configured for scrolling to reach all of the indicators. The UI thus aggregates indicators and, in some cases, other information from the multiple datasets in a way that enables a user, such as a medical professional, to compare this information visually and make an informed decision regarding which, if any, of the potential donors is a good, or the best, candidate to be a donor for the recipient.

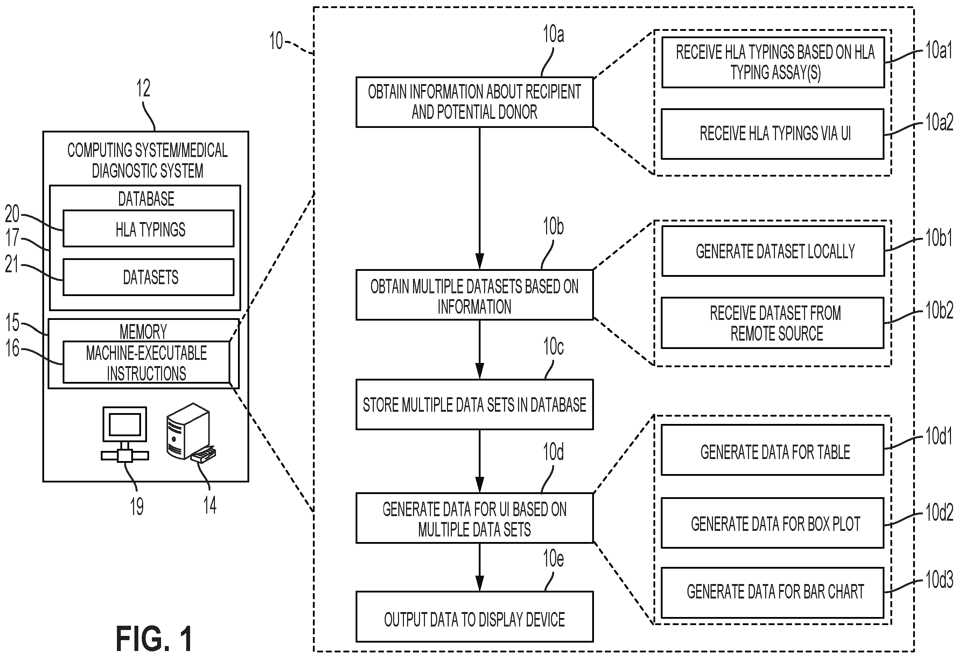

Fig. 1 includes a flowchart showing operations included in an example process 10 for obtaining datasets from multiple sources and for storing the datasets in a manner that enables Uls of the type described herein to be generated.

Process 10 may be performed on, or using, a medical diagnostic system, a generic computing system of which examples are described herein, or a combination of a medical diagnostic system and a generic computing system, any or all of which may be referred to herein as a "system". In the example of Fig. 1, system 12 includes one or more processing devices 14 (e.g., microprocessor(s)) and memory 15 for storing machine-executable instructions 16 that are executable by processing device(s) 14 to perform process 10. Computer storage (e.g., persistent storage such as one or more hard drives(s)) stores database 17 containing the recipient and donor information described herein. System 12 also includes one or more display devices 19 for displaying the Uls generated by process 10, among other information.

Process 10 includes obtaining (10a) information about a recipient of a transplant and information about one or more potential donors for the transplant to the recipient. The information may include an HLA (human leukocyte antigen) typing for the recipient and an HLA typing for each of the potential donors. HLAs include a complex of genes that encode cell-surface proteins that regulate an immune system of a person. Each person's HLA typing includes HLA MHC (major histocompatibility complex) class I (A, B, C) genes and HLA MHC class II (e.g., DR, DQ, DP) genes.

HLA genes are polymorphic, which means that they can have different alleles. An allele is a variant of a sequence of nucleotides of a gene at a particular location, or locus, on a deoxyribonucleic acid (DNA) molecule. The proteins encoded by HLA genes are also referred to as antigens. Each person has a unique set of these antigens, half of which are inherited from the father and half of which are inherited from the mother. The more alike HLA genes of a recipient of a transplant and a potential donor for the transplant are, the more alike the antigens are, and the less likely it is that the transplant from the donor will be rejected by the recipient.

The HLA typings of the recipient and the donor may be obtained using any known HLA typing assay. The HLA typings of the recipient and/or the donor may be results of an HLA typing assay, for example any known HLA typing assay. Examples of such assays may be performed using systems from Werfen^{®} S.A. such as the MIA FORA^{™} NGS MFlex system or the LIFECODES^{®} HLA SSO typing kits.

The HLA typings 20 generated by the assays for the recipient and potential donors may be stored by such a system in database 17 in association with identities of the recipient and potential donors. Alternatively, process 10 may receive (10a1) the HLA typings 20 from such a system and store the HLA typings 20 in database 17 in association with identities of the recipient and potential donors.

Particularly, the HLA typings 20 resulting from the assays for the recipient and/or potential donors may be stored by such a system in database 17 in association with identities of the recipient and potential donors.

In some implementations, process 10 may obtain (10a) the HLA typings by generating and displaying a UI. The UI may contain fields in which the HLA typings of the recipient and the donor may be entered manually by a user. These manually-entered HLA typings may be received (10a2) by process 10 and stored in database 17 in association with identities of the recipient and potential donors.

Process 10 may use the obtained HLA typings of the recipient and potential donors to obtain (10b) multiple datasets 21 and to store the datasets 21 in database 17 as described below. Each dataset may include indicators that are based on a compatibility of a potential donor to the recipient. An indicator may be or include any marking, shading, coloring, value, score, text, icon, picture, symbol, or other indicia that can be used to express the compatibility of a potential donor to the recipient.

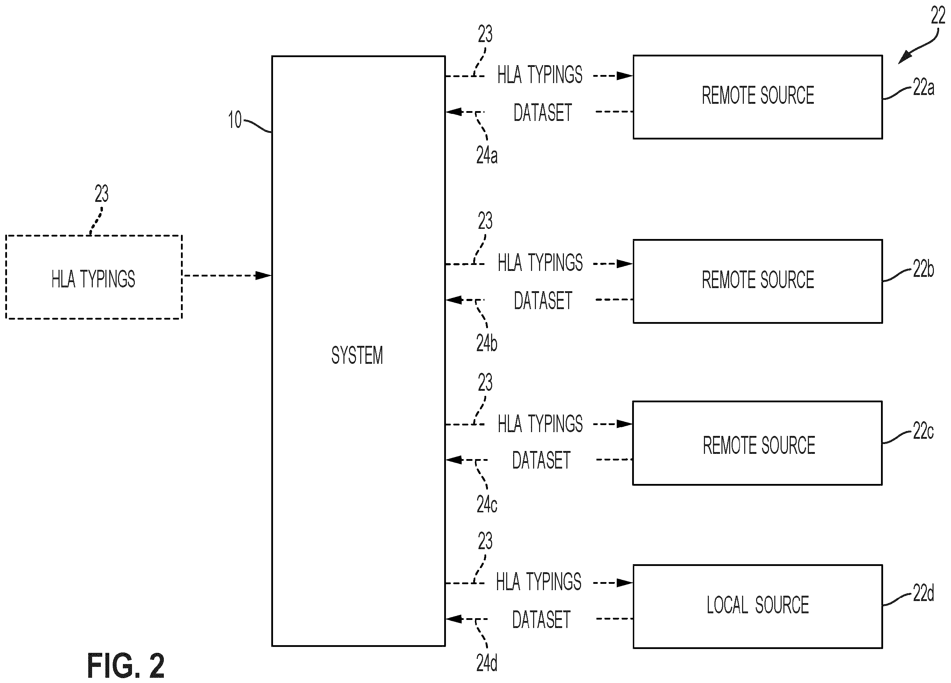

Process 10 may obtain the multiple datasets from multiple different sources. The different sources may include one or more local sources and/or one or more remote sources that are different from the local sources. Fig. 2 illustrates system 12 obtaining datasets from multiple sources 22, including remote sources 22a, 22b, 22c and local source 22d. Although four sources are shown in this example, there may be one, two, three, five, six, seven, or more sources. In these examples, HLA typings 23, which include an HLA typing 20 of a transplant recipient and a potential donor, are sent to a source and the source replies by sending a respective dataset 24a to 24d (which may be part of datasets 21), as described below. In some implementations, in the case of a local source, the local source may receive the HLA typings from the system that performed the assay(s), generate the dataset, and send the dataset to process 10 without first being prompted to do so by process 10.

In some implementations, the local source may receive results of HLA typings, for example from a system which is configured to perform HLA typing assays, and/or from a system which is configured to provide results of HLA typing assays.

A local source may include an entity or system for example operated by, or under control of, the same entity or system that performs the assays to obtain the HLA typings. The local source may generate (10b1) a dataset locally. For example, the local source may perform one or more processes to compare alleles of the recipient to alleles of the potential donors, and generate one or more indicators based on the comparison. The indicators may be any of the indicators described herein, such as shadings, colors, cross-hatchings, or values, that are based on, and indicative of, the difference between alleles of the potential donor and the recipient and, thus, indicative of the compatibility of the potential donor to the recipient for the transplant.

The comparison may be based on the names or designations of the alleles. As explained at "https://hla.alleles.org/nomenclature/naming.html" (accessed August 12, 2024), "Each HLA allele name has a unique number corresponding to up to four sets of digits separated by colons. The length of the allele designation is dependent on the sequence of the allele and that of its nearest relative. All alleles receive at least a four digit name, which corresponds to the first two sets of digits, longer names are only assigned when necessary. The digits before the first colon describe the type, which often corresponds to the serological antigen carried by an allotype. The next set of digits are used to list the subtypes, numbers being assigned in the order in which DNA sequences have been determined. Alleles whose numbers differ in the two sets of digits must differ in one or more nucleotide substitutions that change the amino acid sequence of the encoded protein. Alleles that differ only by synonymous nucleotide substitutions (also called silent or non-coding substitutions) within the coding sequence are distinguished by the use of the third set of digits. Alleles that only differ by sequence polymorphisms in the introns, or in the 5' or 3' untranslated regions that flank the exons and introns, are distinguished by the use of the fourth set of digits. In addition to the unique allele number, there are additional optional suffixes that may be added to an allele to indicate its expression status. Alleles that have been shown not to be expressed - 'Null' alleles - have been given the suffix 'N'. Alleles that have been shown to be alternatively expressed may have the suffix 'L', 'S', 'C', 'A' or 'Q'."

In an example, process 10 may determine, based on comparison(s) of allele designations, which of the alleles of each potential donor at each locus are compatible with corresponding alleles of the recipient at the same locus. Process 10 may store (10c), in database 17, a dataset containing indicators that convey information whether each allele is compatible with a corresponding allele of the recipient at the same locus. For example, for a compatible allele, process 10 may store a label in association with the allele. The label may specify to represent the allele in a certain way on a UI, e.g., as bold, cross-hatched horizontally, or shaded a certain color, such as green, to indicate that the allele is compatible. For example, for a non-compatible allele, process 10 may also store a label in association with the allele. The label may specify to represent the allele in a certain way on a UI, e.g., not bold, cross-hatched vertically, or shaded a certain color, such as red, to indicate that the allele is not compatible. The red/green or other difference in shading is based on comparison of the donor's HLA typing to the recipient's HLA typing. In an example, if there is a match between alleles in the donor and recipient HLA typings, the donor's typing is highlighted green or represented in a first way (e.g., horizontally-hatched). In an example, if there is not a match between alleles in the donor and recipient HLA typings the donor is highlighted red or shaded in a represented way that is different than the first way (e.g., vertically-hatched).

In some implementations, only portions of alleles may be shows in a donor columns. This is based on the resolution in which the donor was typed. A*01:01 would be a lower resolution typing than A*01:01:02 or A*01:01:02:03. The UI allows for a digit allelic (A*01:01) and higher resolutions. In terms of the crossmatching alleles, if there is a resolution mismatch between donor and recipient - for example, the donor allele is shown at a lower resolution than the recipient allele - the process may crossmatch to the lowest resolution. So, for example, A*01:01 with the donor's A*01:01:02:03 would be marked as a match because A*01:01 includes all higher resolutions.

Database 17 may store (10c) datasets 21 in multiple tables. In some implementations, each table includes the identity of the recipient and donor, the information that was used to obtain the dataset, such as the HLA typings of the recipient and the donor, and the information obtained for the dataset. For example, the information obtained for the dataset may include indicators about the compatibility of the donor as a whole to the recipient or of different traits of the donor to the recipient, such as compatibility of individual alleles, T-cells, or B-cells. In some implementations, the information may include labels, such as those described above, in association with alleles of each donor to shade or color each allele based on compatibility to corresponding alleles of the recipient. In some implementations, the labels, may indicate how much shading or what type of shading to apply to each indicator. In some implementations, the information may identify biological features of the recipient and donor that are mismatched, such as alleles and/or eplets.

The remote sources may include entities or systems that are not operated by, or under control of, the same entity or system that generated the HLA typings. To obtain the datasets from the remote sources, process 10 may provide each remote source with information, such as the HLA typings of the recipient and potential donors.

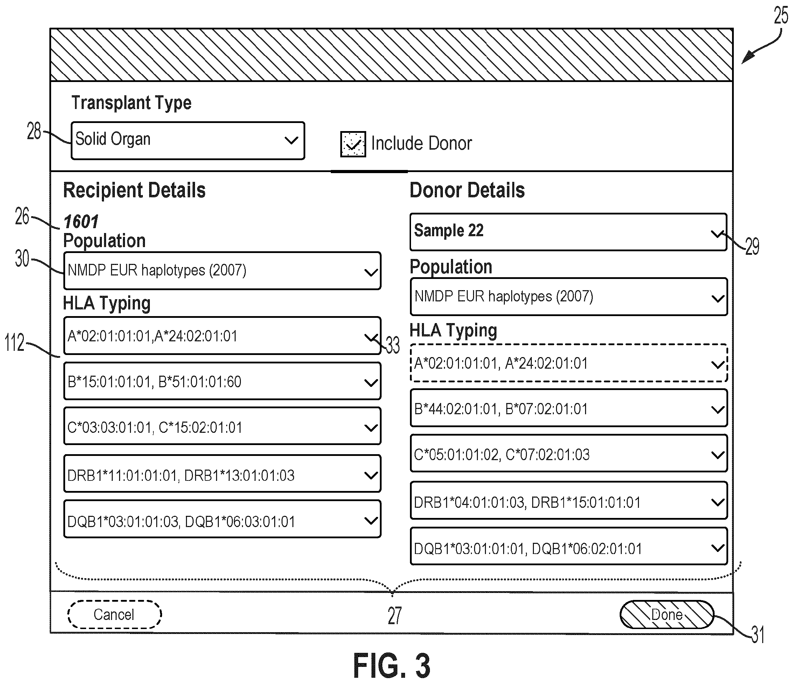

In an example, to obtain datasets from the remote sources, process 10 may generate a window containing fields to populate with HLA typings and, if necessary, other information to provide to the remote source to obtain a dataset from the remote source. For example, referring to Fig. 3, process 10 may generate and display, to a user, window 25 for a pre-designated recipient 26 (#1601).

Window 25 contains fields in which a user and/or system 12 can enter information for the recipient or for the recipient and a potential donor. These fields include HLA typings fields 27 to provide the HLA typings of the recipient or of the recipient and a potential donor. In this example, HLA typings for the recipient may be populated based on the identity of the recipient (here #1601 26). In some implementations, system 12 automatically - for example, without user prompting or input - may retrieve HLA typings of the recipient from database 17, and populate the HLA typings fields 17 fields for the recipient with that information. The dropdown menus (e.g., 33) for the HLA typing fields contain any ambiguities for a locus. The user and/or system can open a dropdown menu and select a different HLA pair. These ambiguities are part of a patient's typing. A user and/or the system may select the pair they wish to use to determine compatibility between recipient and donor. In some implementations, a dropdown menu is configured to be openable by the user for selecting a different HLA pair. In some implementations, the HLA pair which is chosen to determine compatibility between recipient and donor may be configured to be selectable by the user.

The HLA typing fields for the donor may be populated by system 12 in response to specifying the identity of the donor. For example, field 29 include a drop-down box containing a list of potential donors for the recipient. The example donor here is called "Sample 22". In some implementations, the potential donors in the list may include only those donors previously determined not to be incompatible with the recipient or who have a level of compatibility that is greater than a predefined threshold. This may have been determined based, e.g., on the comparison of alleles or other processes performed locally to determine compatibility. In some implementations, the potential donors in the list may include all potential donors who have their HLA typings stored in database 17. A user and/or the system 12 may select a potential donor from the drop-down box. In response to this selection, system 12 automatically may retrieve HLA typings of the selected donor from the database 17, and populate the HLA typings fields 17 for the donor with that information. As above, the user and/or system may open a dropdown menu and select a different HLA pair for the donor.

In some implementations, window 25 may include fields to allow the user and/or system 12 to specify the type of transplant 28, such as stem cell or solid organ, and the population 30 or haplotype of the recipient and the donor.

The system may be configured to provide a selection through a button or prompt 31, which may instruct the system 12 to provide the information from window 25 to one or more sources, wherein the button or prompt 31 is for example operatable by the user. The user, through selection of a button or prompt 31, may instruct the system 12 to provide the information from window 25 to one or more sources. Alternatively, the system may send this information automatically once all necessary fields have been populated. The information from window 25 may be sent electronically over one or more computer networks to a remote source.

In some implementations, the same information may be sent multiple times to multiple different remote sources using the same window or using different windows or other types of Uls than that shown in Fig. 3. Each source may be or include one or more third-party services that receive HLA typings of the recipient and potential donors and that provide a dataset containing indicators, based on the HLA typings, conveying the compatibility between the recipient and donor for a transplant.

Referring back to Fig. 1, process 10 receives (10b2) a dataset from a source in response to providing the source with the HLA typings and, if needed, other information such as the transplant type and population information described above. The dataset received from the source includes indicators of compatibility that are based on factors that can be determined from the HLA typings and/or other information provided, such as, a compatibility of thymus (T-) cells in the donor to T-cells in the recipient, a compatibility of bursa (B-) cells in the donor to B-cells in the recipient, and/or unaligned peptides between the HLA typing of the recipient and the HLA typing of the donor.

In some implementations, process 10 receives (10b2) multiple datasets from multiple different sources that are based on the different factors. For example, process 10 may receive (10b2) a first dataset from a first source that includes indicators of compatibility that are based on T-cells of the donor and recipient, a second dataset from a second source that includes indicators of compatibility that are based on B-cells of the donor and recipient, and a third dataset from a third source that includes indicators of compatibility that are based on unaligned peptides between the HLA typings of the recipient and the donor. In this example, the first, second, and third sources are different from each other, and the first, second, and third datasets are different from each other. For example, the datasets may contain different information, such as different indicators of compatibility.

In some implementations, process 10 receives (10b2) multiple datasets from multiple different sources that are based on the same factor. For example, process 10 may receive (10b2) a first dataset from a first source that includes indicators of compatibility that are based on B-cells of the donor and recipient, a second dataset from a second source that includes indicators of compatibility that are based on the B-cells, and a third dataset from a third source that includes indicators of compatibility that are based on the B-cells. In this example, the first, second, and third sources are different from each other, and the first, second, and third datasets are different from each other. For example, the datasets may contain different information, such as different indicators of compatibility.

Process 10 may receive (10b2) any combination of the foregoing datasets and datasets based on factors not listed herein. Process 10 may obtain datasets from any number of sources. For example, process 10 may obtain data sets from two remote sources, three remote sources, four remote sources, five remote sources, and so forth.

Process 10 stores (10c) the datasets 21 received from each remote source in database 17 so that content from the datasets can be retrieved at a later time. As described above with respect to the local sources, the database may contain multiple tables. In some implementations, each table includes the identity of the recipient and donor, the information that was sent to the remote source, such as the HLA typings of the recipient and the donor and, in some cases, the population and transplant type, and the information received from the remote source. As explained above, the information received from the remote source may include indicators about the compatibility of the donor as a whole to the recipient or of different traits of the donor to the recipient, such as compatibility based on individual alleles, T-cells, or B-cells. In an example, the information may include labels, such as those described above.

The datasets from the local and remote sources are linked in database 17 by the tables. For example, the tables include the identities of the donor and the recipient and the HLA typings of the donor and the recipient. This information is common to all of the tables in some implementations and may be used by the system 12 to access and to retrieve, from database 17, information from any table needed to generate the Uls described herein.

In operation 10d of Fig. 1, process 10 generates data for a UI based on one or more of the datasets 21 stored in database 17. To generate data for the UI, process 10 requests and receives the identities of a recipient and potential donors for the recipient. The identities of the recipient and potential donors may be input to system 12 by a user through a UI (not shown). In some implementations, system 12 may permit the user to select any potential donor who has his or her HLA typings stored in database 17. In some implementations, the system may permit the user to select only those donors previously determined not to be incompatible with the recipient or who have a level of compatibility that is greater than a predefined threshold. This may have been determined, e.g., based on the comparison of alleles described above. In some implementations, the system 12 may be configured to permit the user to select any potential donor who has his or her HLA typings stored in database 17. In some implementations, the system may be configured to permit the user to select only those donors previously determined not to be incompatible with the recipient or who have a level of compatibility that is greater than a predefined threshold.

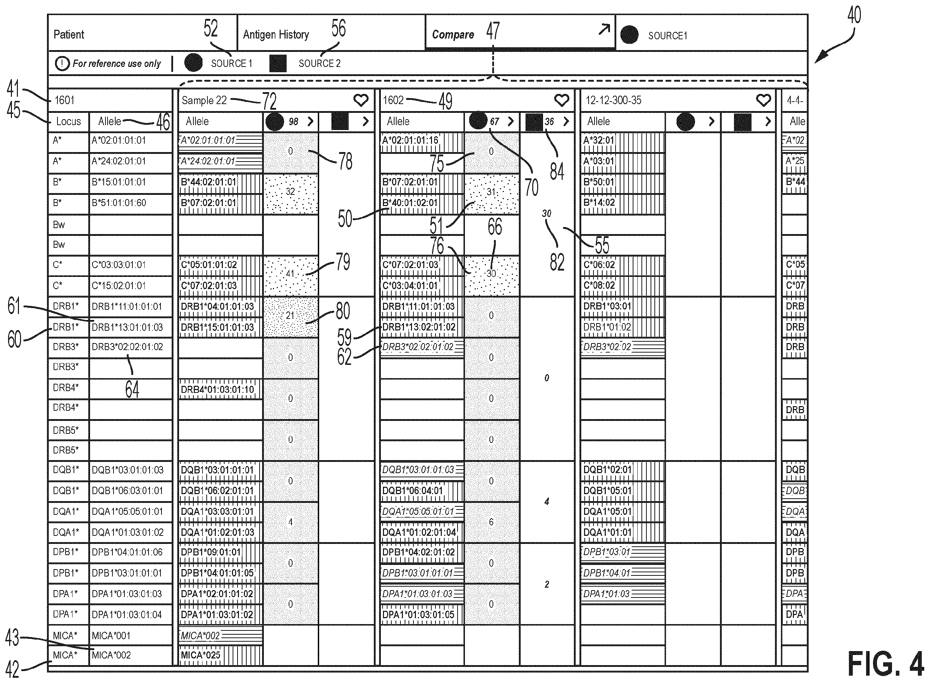

Fig. 4 shows an example of a UI 40 that may be generated (10d) according to process 10. In this example, UI 40 is a table (10d1) that includes information and indicators from datasets that were generated locally and datasets that were received from two remote sources. The information to generate the data for UI 40 is stored in database 17 as described above, and retrieved from the database to generate the UI.

UI 40 includes information about a recipient 41 in columns 42 and 43. The information includes loci (serotype or serotype/serology) 45 and alleles 46 for the recipient, with each locus and allele at that locus being in the same row.

UI 40 includes information 47 about each potential donor. The sources SOURCE1 52 "•" and SOURCE2 56 "▪" of information for each potential donor are the same in this example. In some implementations, the sources may be different for different donors, e.g., if one source did not provide information for a donor, information from the source may be missing from UI 40. In this example, the type of information for each donor is the same, although the content of that information is different for different donors. Accordingly, the information for donor 49 is described primarily.

UI 40 includes information 50 from a local source, information 51 from a first remote source 52 (SOURCE1), and information 55 from a second remote source 56 (SOURCE2). The first remote source is different from the second remote source. The information from all three sources includes indicators that express compatibility of the donor to the recipient based on different factors and in different ways.

In this example, the information 50 from the local source includes the alleles for the potential donor at the loci 45 specified for the recipient. So, for example, allele 59 is at locus 60 in the potential donor and allele 61 is at the same locus 60 in the recipient. In this example, allele 59 is shaded (e.g., red or vertically-hatched) to indicate that allele 59 is different from allele 61. In another example, allele 62 is shaded (e.g., green or horizontally-hatched) to indicate that allele 62 is the same as allele 64. Alleles that are the same in both a recipient and donor are indicators of compatibility, whereas alleles that are different in the recipient and donor are indicators of incompatibility.

In this example, information 51 from remote source 52 includes indicators of compatibility that are based on T-cells of the donor and recipient. In this example, the indicators include values, with a value 66 being assigned to each antigen (combined alleles), and with a composite value 70 being assigned to the donor. In this example, the composite value 70 is a sum of individual antigen values "30", "31", and "6". In the example of Fig. 4, donor 49 has a composite value of "67" and donor 72 has a composite value of "98". This means that, for a transplant under consideration, in the context of T-cell compatibility, donor 49 is more compatible with recipient 41 than donor 72, since donor 49's composite value is less than donor 72's composite value. There is a similar relationship for the individual antigen values. For example, antigen 75 is more compatible with the corresponding recipient antigen than is antigen 76.

In this example, the indicators also include shadings associated with each antigen. For example, in the context of T-cell compatibility, darker shadings indicate greater compatibility than lighter shadings. In the example of donor 72, antigen 78 is shaded darkest meaning, in the context of T-cell compatibility, antigen 78 has relatively good compatibility with the recipient. Antigen 79 is shaded lightest meaning, in the context of T-cell compatibility, antigen 79 has relatively bad compatibility with the recipient, at least compared to the other antigens listed. Antigen 80 has an amount of shading between that of antigens 78 and 79 which, in the context of T-cell compatibility, means compatibility that antigen 80 has better compatibility with the recipient than antigen 79, but not as good compatibility with the recipient as antigen 78.

In this example, information 55 from remote source 56 includes indicators of compatibility that are based on B-cells of the donor and recipient. In this example, the indicators include values, with a value 82 being based on the loci or serotype of the recipient and donor and with a composite value 84 being assigned to the donor. In this example, the composite value is a sum of the individual values 82; however, other implementations may generate a composite value through other combinations of the allele values. The composite value may be a number of eplet mismatches in B-cells for the recipient and donor. In the example of Fig. 4, donor 49 has a composite value of "36", which is a sum of the "30", "4", and "2" values shown in the column. In general, a lower value indicates greater compatibility while a higher value indicates less compatibility.

In some implementations, process 10 may generate the composite values locally. In some implementations, the composite values may be part of the dataset transferred from the local or remote source. In some implementations, process 10 may combine indicators, such as values from different sources, to generate additional indicators, and present those indicators on locations of the UI.

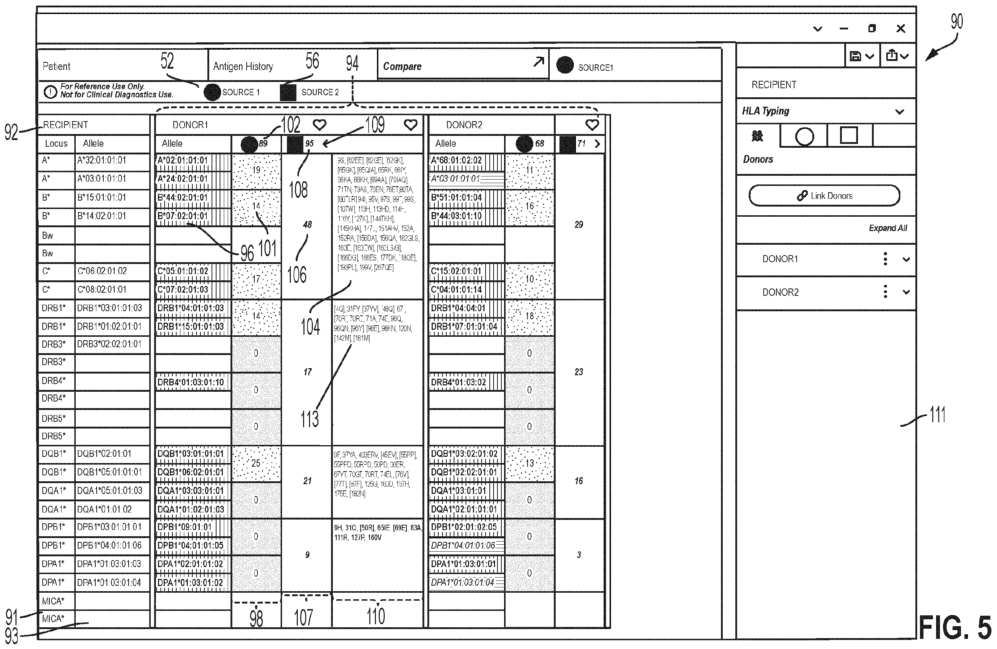

Fig. 5 shows another example of a UI 90 that may be generated according to process 10. In this example, UI 90 is a table (10d1) that includes information and indicators from datasets that were generated locally and datasets that were received from two remote sources. The information to generate the data for UI 90 is stored in database 17 as described above, and retrieved from the database to generate the UI.

UI 90 includes information about a recipient 92 in columns 91 and 93. The information includes loci (serotype or serotype/serology) and alleles for the recipient, with each locus and allele at that locus being the same row. This information may be obtained in operation 10a using the techniques described herein. UI 90 is similar to UI 40 in that columns 91 and 93 provide the same type of information about the recipient as columns 42 and 43 of Fig. 4.

UI 90 includes information about each potential donor (DONOR1, DONOR2,). The sources of information (SOURCE 1 and SOURCE 2) for each potential donor are the same as with respect to UI 40. In addition, the type of information for each donor is the same, although the content of that information for different donors is different. Accordingly, only the information for donor 95 (DONOR1) is described.

In this example, the information 96 from local source includes the alleles for the potential donor at the loci specified for the recipient. The information may be the same type of information as, and presented in the same manner as, that described with respect to UI 40.

In this example, information 98 from remote source 52 includes indicators of compatibility that are based on T-cells of the donor and recipient. In this example, the indicators include antigen values, with a value 101 being assigned to each antigen (combined alleles). In this example, the values include a composite value 102 assigned to the donor, which may be based on a combination of the allele and/or antigen values as described with respect to UI 40. As was the case above, the lower the values are in this example, the more likely that the donor is compatible with the recipient. That is, lower values indicate greater compatibility with their counterparts on the recipient.

The indicators also include shadings associated with each allele and with each antigen. In this example, the shadings have the same meanings described with respect to UI 40. That is, darker shadings indicate greater compatibility than lighter shadings.

In this example, information 104 from remote source 56 includes indicators of compatibility that are based on B-cells of the donor and recipient. In this example, the indicators include values, with a value 106 being based on the loci or serotype of the recipient and donor and with a composite value 108 being assigned to the donor. In this example, the composite value is a sum of the individual values 106; however, other implementations may generate a composite value through other combinations of the allele values. The composite value may be a number of eplet mismatches in B-cells for the recipient and donor. In general, a lower value indicates greater compatibility while a higher value indicates less compatibility.

UI 90 also includes arrow 109 that is selectable by a user to cause column 107 to expand column to display column 110. Column 110 lists, for combinations of alleles, the eplets 113 that were mismatched in the B-cells between the donor and the recipient.

In some implementations, UI 90 may rank the donors in terms of compatibility to the recipient. The ranking may be displayed as a list in area 111 of UI 90, with the donor at the top of the list being most compatible with the recipient, and successively lower-listed donors being less compatible. Process 10 may determine the rankings by assessing the indicators provided by each source for each donor. The donor with the fewest negative indicators of compatibility may be ranked as highest - that is, most compatible to recipient for the transplant. Lower-ranked - that is, lower listed - donors have successively greater negative indicators of compatibility with the recipient.

Referring back to Fig. 1, process 10 outputs (10e) data for to a display device, such as display device 19, to display the UI. A medical professional may review the UI and, if necessary, scroll through the UI, to make a decision about which, if any, of the potential donors is a good candidate. In some implementations, the UI may be configured to allow a user, such as a medical professional, to review the UI and, if necessary, allow to scroll through the UI, for making a decision about which, if any, of the potential donors is a good candidate. Having all of the information in a single location, such as the Uls described herein, may be advantageous since it reduces the need for a medical professional to navigate different UIs and to access different sources to obtain the information. Furthermore, the Uls described herein consolidate the information from different sources and convey the information in a manner that enables information from the different sources to be compared easily.

Referring to Figs. 1 and 3, in some implementations system 12 may provide a source with an HLA typing 112 of the recipient only (that is, no donor HLA typing) and a population (e.g., haplotype) containing the recipient. With this information and without the HLA typing of the donor, the source returns a dataset containing information about the population. This information may be used to generate (10d2, Fig. 1) a box plot.

The box plot represents a range of indicators from the source - in this example, values - for the population that are based on compatibility of members of the population to the recipient for the transplant. In this example, lower values indicate greater levels of compatibility. The values may be composite values generated by the source for random members of the population. Examples of such composite values are described with respect to Uls 40 and 90.

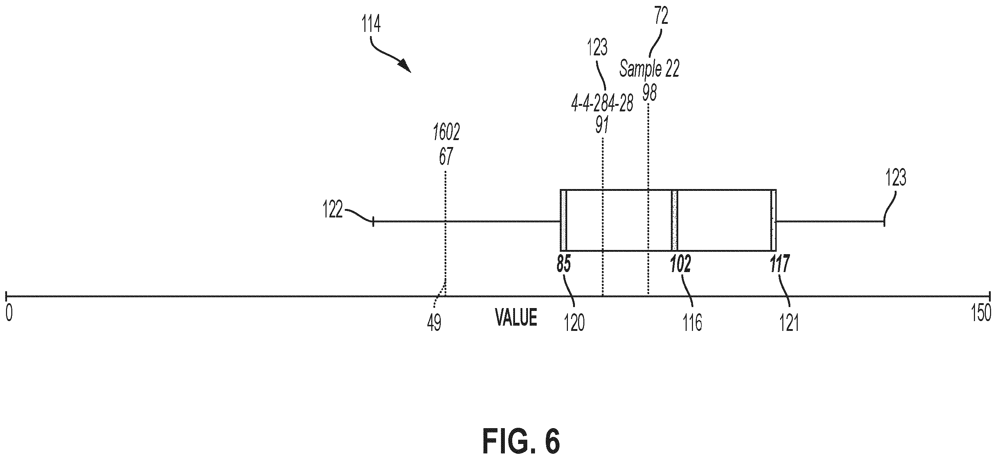

Referring to the box plot 114 of Fig. 6, in an example, the range includes an average value 116 of "102". Value 116 is the average of compatibility values for members in the population for compatibility to the recipient for the transplant. The range includes a first percentage, e.g., first quartile value 120. Value 120 ("85") means that 25% of the population have a greater compatibility to the recipient for the transplant. The range includes a second percentage, e.g., third quartile value 121 ("117"). Value 121 means that 75% of the population have a greater compatibility to the recipient for the transplant. The range also includes third 122 and fourth 123 percentage values that mean, respectively, that 5% of the population have a greater compatibility to the recipient for the transplant and that 95% the population have a greater for compatibility to the recipient for the transplant. In some implementations, third 122 and fourth 123 values are not shown on the box plot but rather are represented graphically by lines.

To generate (10d2) data for a UI containing the box plot, in this example, process 10 retrieves, from database 17, indicators from the same source providing data used to generate the box plot. In this example, process 10 accesses database 17 for to obtain composite values (indicators) for potential donors. The composite values for the potential donors may be based on compatibility of the multiple potential donors to the recipient for the transplant, as described herein. In some implementations, the system may permit composite values to be obtained for any potential donor who has their HLA typings stored in the database. In some implementations, the system may permit composite values to be obtained for only those donors previously determined not to be incompatible with the recipient or who have a level of compatibility that is greater than a predefined threshold. This may have been determined, e.g., based on the comparison of alleles described above.

Process 10 overlays the composite values for the potential donors at appropriate locations on the box plot. For example, as shown in Fig. 6, value "67" for donor 49 (Fig. 4) is overlaid at below the first quartile 120; value "91" for donor 123 is overlaid between the first quartile 120 and the average 116; and value "98" for donor 72 (Fig. 4) is overlaid between the first quartile 120 and the average 116 but above the value for donor 123. What the resulting graphic shows is that, among the potential donors, donor 49 with a value of "67" has the lowest value and, therefore, among potential donors, is the best candidate for the transplant. Furthermore, since fewer than 25% of people in the population are likely to have a better value than donor 49, donor 49 is a good candidate for the transplant within the population.

In some implementations, process 10 may generate plots other than a box plot, such as histograms, violin plots, strip plots, swarm scatter charts, line charts, or pie charts relating values for potential donors to a population in order to show which of the potential donors is the best candidate for a transplant to a recipient.

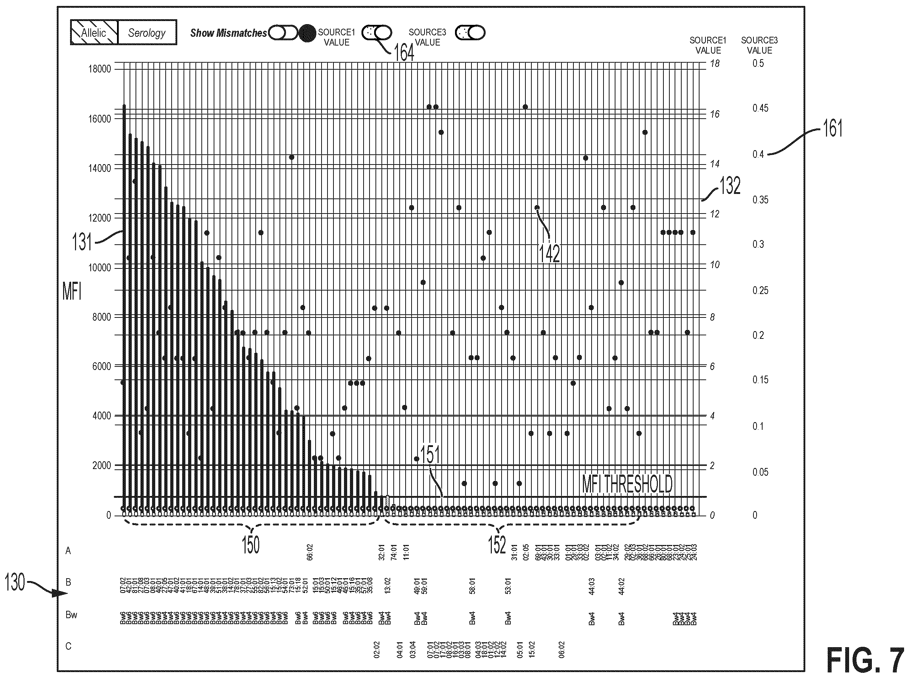

Referring to Figs. 1 and 7, in some implementations, a dataset returned by a source, such as a local source, may include MFI (mean fluorescence intensity) values. The MFI values are based on antigens of the recipient that are indicative of rejection of the transplant from the potential donor. For example, an assay may contain beads, each containing an antigen. When a sample from the recipient, such as blood, contacts the bead, if the blood has an antibody for the antigen, the bead fluoresces. The greater the fluorescence is, the stronger the reaction is between the sample and the antigen. The MFI values reflect the strength of the reaction for each antigen/bead. The greater the MFI value is, the less compatible the recipient is with a donor that has that antigen.

Process 10 may generate (10d3, Fig. 1) data for a single graphic, such as bar chart, that combines the MFI values with indicators of compatibility from one or more other sources. For example, process 10 combine values for alleles or antigens, such as values 66 of Fig. 4 or values 101 of Fig. 5, from one source (e.g., a remote source) with MFI values from a different source (e.g., a local source). In the bar chart of Fig. 7, axis 130 corresponds to antigens. Axis 131 lists MFI values, with the length of the bars indicating the magnitude of the MFI values. MFI values over a predefined threshold may be shaded, colored (e.g., pink), or cross-hatched differently from MFI values below the threshold, which may be shaded, colored (e.g., green), or cross-hatched to identify which MFI values are incompatible with a transplant from a donor to the recipient and which MFI values are compatible with a transplant from the donor to the recipient. Bars 150 denote positive antigens (the MFI value is above the MFI threshold 151) and bars 152 are negative antigens (the MFI values are below the MFI threshold). In an example, the MFI threshold may be set to 750.

Process 10 combines the values for alleles/antigens, such as values 101 of Fig. 5, from a source that may be different from the source that provided the MFI values or from a source that is the same source that provided the MFI values. In this example, axis 132 includes a range of values from a source such as source 52 of Fig. 4 and the dots, such as dots 142, represent a value of an antigen from that source. In this example, axis also 132 includes a range of values from a different source 160 (labeled SOURCE3), which correspond to the values of SOURCE1. Tabs 164 allow a user to select which value or values (from SOURCE1 or SOURCE3) to display. In some implementations, tabs 164 are configured to allow a user to select which value or values, for example from SOURCE1 and/or SOURCE3, to display.

Integrating source values such as those described above with MFI values allows for a more nuanced understanding of immunogenic risk by combining both the theoretical risk (the source value) and the practical measure of antibody binding strength (MFI). This combined approach helps assess not just the risk but also the potential impact of that risk based on how strongly the antibodies attached to antigens.

All or part of the systems and processes described in this specification and their various modifications may be executed or controlled at least in part by one or more computers such as system 12 using one or more computer programs tangibly embodied in one or more information carriers, such as in one or more non-transitory machine-readable storage media. A computer program can be written in any form of programming language, including compiled or interpreted languages, and it can be deployed in any form, including as a stand-alone program or as a module, part, subroutine, or other unit suitable for use in a computing environment. A computer program can be deployed to be executed on one computer or on multiple computers at one site or distributed across multiple sites and interconnected by a network.

Actions associated with executing the processes or controlling the test system described herein can be performed by one or more programmable processors executing one or more computer programs to control or to perform all or some of the operations described herein. All or part of the test systems and processes can be executed or controlled by special purpose logic circuitry, such as, an FPGA (field programmable gate array) and/or an ASIC (application-specific integrated circuit) or embedded microprocessor(s) localized to the instrument hardware.

Processors suitable for the execution of a computer program include, by way of example, both general and special purpose microprocessors, and any one or more processors of any kind of digital computer. Generally, a processor will receive instructions and data from a read-only storage area or a random-access storage area or both. Elements of a computer include one or more processors for executing instructions and one or more storage area devices for storing instructions and data. Generally, a computer will also include, or be operatively coupled to receive data from, or transfer data to, or both, one or more machine-readable storage media, such as mass storage devices for storing data, such as magnetic, magneto-optical disks, or optical disks. Non-transitory machine-readable storage media suitable for embodying computer program instructions and data include all forms of non-volatile storage area, including by way of example, semiconductor storage area devices, such as EPROM (erasable programmable read-only memory), EEPROM (electrically erasable programmable read-only memory), and flash storage area devices; magnetic disks, such as internal hard disks or removable disks; magneto-optical disks; and CD-ROM (compact disc read-only memory) and DVD-ROM (digital versatile disc read-only memory).

In particular embodiments, the method and in particular its steps as disclosed herein, and/or the operations of the one or more non-transitory machine-readable storage devices storing instructions that are executable by one or more processing devices to perform said operations are not performed on the human or animal body by surgery or therapy and diagnostic methods practised on the human or animal body.

As used herein, the terms "comprises," "comprising," "includes," "including," "has," "having," "contains," "containing," and any variations thereof, are intended to cover a non-exclusive inclusion, such that systems, techniques, apparatus, structures, processes, or other subject matter described or claimed herein that includes, has, or contains an element or list of elements does not include only those elements but can include other elements not expressly listed or inherent to such systems, techniques, apparatus, structures, processes or other subject matter described or claimed herein.

All examples described herein are non-limiting.

In the description and claims provided herein, the adjectives "first", "second", "third", and the like do not designate priority or order unless context suggests otherwise. Instead, these adjectives may be used solely to differentiate the nouns that they modify.

Elements of different implementations described may be combined to form other implementations not specifically set forth previously. Elements may be left out of the systems described previously without adversely affecting their operation or the operation of the system in general. Furthermore, various separate elements may be combined into one or more individual elements to perform the functions described in this specification.

Other implementations not specifically described in this specification are also within the scope of the following claims.

Further aspects, features and embodiments of the present invention are described in the following items:
1. A method performed by one or more processing devices, comprising:
   obtaining information about a recipient of a transplant and a potential donor of the transplant, the information comprising an HLA (human leukocyte antigen) typing for the recipient and an HLA typing for the potential donor;
   obtaining multiple datasets based on the information, each dataset comprising indicators that are based on a compatibility of the potential donor to the recipient for the transplant;
   storing the multiple data sets in database;
   generating data for a user interface (UI) containing representations of the indicators from ones of the multiple datasets in the database; and
   outputting the data for display on a display device.
2. A method performed by one or more processing devices, comprising:
   providing information about a recipient of a transplant and a potential donor of the transplant, the information comprising an HLA (human leukocyte antigen) typing for the recipient and an HLA typing for the potential donor;
   obtaining and/or providing multiple datasets based on the information, each dataset comprising indicators that are based on a compatibility of the potential donor to the recipient for the transplant;
   storing the multiple data sets in database;
   generating data for a user interface (UI) containing representations of the indicators from ones of the multiple datasets in the database; and
   outputting the data for display on a display device.
3. The method of item 1 or 2, wherein the indicators are based on compatibility of thymus (T-) cells in the donor to T-cells in the recipient.
4. The method of any one of the items 1 to 3, wherein the indicators are based on compatibility of bursa (B-) cells in the donor to B-cells in the recipient.
5. The method of any one of the items 1 to 4, wherein the indicators are based on unaligned peptides between the HLA typing of the recipient and the HLA typing of the donor.
6. The method of any one of the items 1 to 5, wherein the datasets are from different sources, the different sources comprising a local source and a remote source, the local source comprising an entity from which the information was obtained and the remote source being different from the entity from which the patient information was obtained.
7. The method of any one of the items 1 to 6, wherein the datasets are from different sources, the different sources comprising a local source and a remote source, the local source comprising an entity from which the information is provided and the remote source being different from the entity from which the patient information was obtained.
8. The method of any one of the items 1 to 7, wherein obtaining the information comprises receiving results of HLA typing assays performed on biological samples from the recipient and the potential donor.
9. The method of any one of the items 1 to 8, wherein providing the information comprises receiving results of HLA typing assays performed on biological samples from the recipient and the potential donor.
10. The method of any one of the items 1 to 9, wherein providing the information comprises providing results of HLA typing assays performed on biological samples from the recipient and the potential donor.
11. The method of any one of the items 1 to 10, wherein the UI comprises a table, the table comprising columns for the recipient and columns for the potential donor, the columns for the recipient comprising a first column containing loci and a second column containing alleles of the recipient at corresponding loci in the first column, and the columns for the potential donor comprising a third column containing alleles of the potential donor at corresponding loci in the first column and one or more additional columns containing indicators that are based one or more of a compatibility of thymus (T-) cells in the donor to T-cells in the recipient or a compatibility of bursa (B-) cells in the donor to B-cells in the recipient.
12. The method of item 11, wherein the one or more additional indicators are at rows corresponding to loci and alleles of the recipient or rows corresponding to one or more serotype groups of the recipient.
13. The method of any one of the items 1 to 12, wherein the indicators comprise values representing compatibility of the potential donor to the recipient for the transplant; and
   wherein UI comprises:
   a box plot of a population that includes the recipient, the box plot representing a range of the values for the population that are based on compatibility of members of the population to the recipient for the transplant; and
   values for multiple potential donors overlaid on the box plot, the values for multiple potential donors being based on compatibility of the multiple potential donors to the recipient for the transplant.
14. The method of any one of the items 1 to 13, wherein the multiple datasets comprises MFI (mean fluorescence intensity) values, the MFI values being based on antigens of the recipient that are indicative of rejection of the transplant from the potential donor.
15. The method of item 14, wherein the data for the UI comprises a bar chart containing the MFI values associated with the antigens of the recipient.
16. The method of item 15, wherein the bar chart comprises first axes for the MFI values and indicators and a second axis corresponding to antigens of the recipient that are indicative of rejection of the transplant from the potential donor.
17. The method of any one of the items 1 to 16, wherein the UI comprises a single window that displays all of the indicators or that is configured for scrolling to reach all indicators.
18. The method of any one of the items 1 to 17, wherein storing the multiple data sets in the database comprises storing each dataset in a table, each of the tables including common information comprising an identity of the recipient, an identity of the potential donor, an HLA typing of the recipient, and an HLA typing of the potential donor.
19. One or more non-transitory machine-readable storage devices storing instructions that are executable by one or more processing devices to perform operations comprising:
   obtaining information about a recipient of a transplant and a potential donor of the transplant, the information comprising an HLA (human leukocyte antigen) typing for the recipient and an HLA typing for the potential donor;
   obtaining multiple datasets based on the information, each dataset comprising indicators that are based on a compatibility of the potential donor to the recipient for the transplant;
   storing the multiple data sets in database;
   generating data for a user interface (UI) containing representations of the indicators from ones of the multiple datasets in the database; and
   outputting the data for display on a display device.
20. One or more non-transitory machine-readable storage devices storing instructions that are executable by one or more processing devices to perform operations comprising:
   providing information about a recipient of a transplant and a potential donor of the transplant, the information comprising an HLA (human leukocyte antigen) typing for the recipient and an HLA typing for the potential donor;
   obtaining and/or providing multiple datasets based on the information, each dataset comprising indicators that are based on a compatibility of the potential donor to the recipient for the transplant;
   storing the multiple data sets in database;
   generating data for a user interface (UI) containing representations of the indicators from ones of the multiple datasets in the database; and
   outputting the data for display on a display device.
21. The one or more non-transitory machine-readable storage devices of item 19 or 20, wherein storing the multiple data sets in the database comprises storing each dataset in a table, each of the tables including common information comprising an identity of the recipient, an identity of the potential donor, an HLA typing of the recipient, and/or an HLA typing of the potential donor.
22. The one or more non-transitory machine-readable storage devices of any one of the items 19 to 21, wherein the UI comprises a table, the table comprising columns for the recipient and columns for the potential donor, the columns for the recipient comprising a first column containing loci and a second column containing alleles of the recipient at corresponding loci in the first column, and the columns for the potential donor comprising a third column containing alleles of the potential donor at corresponding loci in the first column and one or more additional columns containing indicators that are based one or more of a compatibility of thymus (T-) cells in the donor to T-cells in the recipient or a compatibility of bursa (B-) cells in the donor to B-cells in the recipient.
23. The one or more non-transitory machine-readable storage devices of any one of the items 19 to 22, wherein the indicators comprise values representing compatibility of the potential donor to the recipient for the transplant; and
   wherein UI comprises:
   a box plot of a population that includes the recipient, the box plot representing a range of the values for the population that are based on compatibility of members of the population to the recipient for the transplant; and
   values for multiple potential donors overlaid on the box plot, the values for multiple potential donors being based on compatibility of the multiple potential donors to the recipient for the transplant.
24. The one or more non-transitory machine-readable storage devices of any one of the items 19 to 23, wherein the multiple datasets comprises MFI (mean fluorescence intensity) values, the MFI values being based on antigens of the recipient that are indicative of rejection of the transplant from the potential donor.
25. The one or more non-transitory machine-readable storage devices of item 24, wherein the data for the UI comprises a bar chart containing the MFI values associated with the antigens of the recipient.
26. One or more non-transitory machine-readable storage devices storing instructions that are executable by one or more processing devices to perform operations comprising the steps of any one of the items 1 to 18.

## Claims

1. A method performed by one or more processing devices, comprising:
obtaining information about a recipient of a transplant and a potential donor of the transplant, the information comprising an HLA (human leukocyte antigen) typing for the recipient and an HLA typing for the potential donor;
obtaining multiple datasets based on the information, each dataset comprising indicators that are based on a compatibility of the potential donor to the recipient for the transplant;
storing the multiple data sets in database;
generating data for a user interface (UI) containing representations of the indicators from ones of the multiple datasets in the database; and
outputting the data for display on a display device.

2. The method of claim 1, wherein the indicators are based on compatibility of thymus (T-) cells in the donor to T-cells in the recipient.

3. The method of claim 1 or 2, wherein the indicators are based on compatibility of bursa (B-) cells in the donor to B-cells in the recipient.

4. The method of any one of the claims 1 to 3, wherein the indicators are based on unaligned peptides between the HLA typing of the recipient and the HLA typing of the donor.

5. The method of any one of the claims 1 to 4, wherein the datasets are from different sources, the different sources comprising a local source and a remote source, the local source comprising an entity from which the information was obtained and the remote source being different from the entity from which the patient information was obtained.

6. The method of any one of the claims 1 to 5, wherein obtaining the information comprises receiving results of HLA typing assays performed on biological samples from the recipient and the potential donor.

7. The method of any one of the claims 1 to 6, wherein the UI comprises a table, the table comprising columns for the recipient and columns for the potential donor, the columns for the recipient comprising a first column containing loci and a second column containing alleles of the recipient at corresponding loci in the first column, and the columns for the potential donor comprising a third column containing alleles of the potential donor at corresponding loci in the first column and one or more additional columns containing indicators that are based one or more of a compatibility of thymus (T-) cells in the donor to T-cells in the recipient or a compatibility of bursa (B-) cells in the donor to B-cells in the recipient,
wherein optionally, the one or more additional indicators are at rows corresponding to loci and alleles of the recipient or rows corresponding to one or more serotype groups of the recipient.

8. The method of any one of the claims 1 to 7, wherein the indicators comprise values representing compatibility of the potential donor to the recipient for the transplant; and
wherein UI comprises:
a box plot of a population that includes the recipient, the box plot representing a range of the values for the population that are based on compatibility of members of the population to the recipient for the transplant; and
values for multiple potential donors overlaid on the box plot, the values for multiple potential donors being based on compatibility of the multiple potential donors to the recipient for the transplant.

9. The method of any one of the claims 1 to 8, wherein the multiple datasets comprises MFI (mean fluorescence intensity) values, the MFI values being based on antigens of the recipient that are indicative of rejection of the transplant from the potential donor,
wherein optionally, the data for the UI comprises a bar chart containing the MFI values associated with the antigens of the recipient,
wherein further optionally, the bar chart comprises first axes for the MFI values and indicators and a second axis corresponding to antigens of the recipient that are indicative of rejection of the transplant from the potential donor.

10. The method of any one of the claims 1 to 9, wherein the UI comprises a single window that displays all of the indicators or that is configured for scrolling to reach all indicators, and/or
wherein storing the multiple data sets in the database comprises storing each dataset in a table, each of the tables including common information comprising an identity of the recipient, an identity of the potential donor, an HLA typing of the recipient, and an HLA typing of the potential donor.

11. One or more non-transitory machine-readable storage devices storing instructions that are executable by one or more processing devices to perform operations comprising:
obtaining information about a recipient of a transplant and a potential donor of the transplant, the information comprising an HLA (human leukocyte antigen) typing for the recipient and an HLA typing for the potential donor;
obtaining multiple datasets based on the information, each dataset comprising indicators that are based on a compatibility of the potential donor to the recipient for the transplant;
storing the multiple data sets in database;
generating data for a user interface (UI) containing representations of the indicators from ones of the multiple datasets in the database; and
outputting the data for display on a display device.

12. The one or more non-transitory machine-readable storage devices of claim 11, wherein storing the multiple data sets in the database comprises storing each dataset in a table, each of the tables including common information comprising an identity of the recipient, an identity of the potential donor, an HLA typing of the recipient, and an HLA typing of the potential donor.

13. The one or more non-transitory machine-readable storage devices of claim 11 or 12, wherein the UI comprises a table, the table comprising columns for the recipient and columns for the potential donor, the columns for the recipient comprising a first column containing loci and a second column containing alleles of the recipient at corresponding loci in the first column, and the columns for the potential donor comprising a third column containing alleles of the potential donor at corresponding loci in the first column and one or more additional columns containing indicators that are based one or more of a compatibility of thymus (T-) cells in the donor to T-cells in the recipient or a compatibility of bursa (B-) cells in the donor to B-cells in the recipient.

14. The one or more non-transitory machine-readable storage devices of any one of the claims 11 to 13, wherein the indicators comprise values representing compatibility of the potential donor to the recipient for the transplant; and
wherein UI comprises:
a box plot of a population that includes the recipient, the box plot representing a range of the values for the population that are based on compatibility of members of the population to the recipient for the transplant; and
values for multiple potential donors overlaid on the box plot, the values for multiple potential donors being based on compatibility of the multiple potential donors to the recipient for the transplant.

15. The one or more non-transitory machine-readable storage devices of any one of the claims 11 to 14, wherein the multiple datasets comprises MFI (mean fluorescence intensity) values, the MFI values being based on antigens of the recipient that are indicative of rejection of the transplant from the potential donor,
wherein optionally, the data for the UI comprises a bar chart containing the MFI values associated with the antigens of the recipient.
